# EUROPÄISCHE PATENTSCHRIFT

(11) **EP 2 865 415 B1**
(45) Veröffentlichungstag und Bekanntmachung des Hinweises auf die Patenterteilung: **31.08.2016**
(21) Anmeldenummer: 14188787.7
(22) Anmeldetag: 14.10.2014
(51) Int. Cl.: A61N 1/39, A61N 1/36, A61N 1/362, A61N 1/37

(54) **Wahrnehmungseinheit für einen Gewebestimulator**
Sensing unit for a tissue stimulator
Unité de perception pour un stimulateur tissulaire

(30) Priorität: 28.10.2013 US 201361896138 P
(43) Veröffentlichungstag der Anmeldung: 29.04.2015
(73) Patentinhaber: BIOTRONIK SE & Co. KG, 12359 Berlin (DE)
(72) Erfinder: Tscherch, Frank, 15741 Bestensee (DE); Kreidler, Christian, 12159 Berlin (DE); Kiefer, Thomas, 12207 Berlin (DE)
(74) Vertreter: Galander, Marcus

(56) Entgegenhaltungen:
- EP-A1- 2 294 978
- US-A1- 2007 146 189
- US-A1- 2013 030 486

## Beschreibung

Die Erfindung betrifft eine Wahrnehmungseinheit zur Verarbeitung elektrischer Signale eines menschlichen oder tierischen Körpers. Derartige Wahrnehmungseinheiten sind Bestandteil von Gewebestimulatoren wie Herzschrittmacher, Defibrillatoren, Cardioverter, Neurostimulatoren und dergleichen und dienen der Verarbeitung elektrischer Signale des Körpergewebes, die üblicherweise mittels Elektroden am, auf oder im Gewebe erfasst werden. Die Wahrnehmungseinheit ist über einen Elektrodenanschluss mit einer solchen Elektrode verbunden und beinhaltet zumindest einen Analog-Digital-Wandler (A/D-Wandler) und eine digitale Filterstufe. Die weitere Verarbeitung der Ausgangssignale der Wahrnehmungseinheit umfasst zumindest eine Bewertung der Signalamplitude, so werden beispielsweise elektrische Signale des Körpergewebes mit einer Amplitude oberhalb eines bestimmten Schwellwertes als natürliche Aktivität erkannt und bestimmten physiologischen Ereignissen anhand ihrer Charakteristik zugeordnet. Zur Stimulation des Körpergewebes enthält der Gewebestimulator eine Stimulationseinheit, über die ein oder mehrere elektrische Impulse über die Elektrode an das Körpergewebe abgegeben werden. Da die Amplitude der Stimulationsimpulse üblicherweise ein Mehrfaches der Amplituden der natürlichen elektrischen Gewebeaktivität beträgt, wird mindestens für die Dauer der Stimulation die Verbindung der Wahrnehmungseinheit zur Elektrode unterbrochen (z.B. bei einer Stimulationsimpulsdauer von 1ms kann diese Zeit der Trennung der Wahrnehmungseinheit von der Elektrode 8 bis 20ms betragen). Stimulationsimpulse verursachen an der Elektrode im Elektrolyten bzw. dem umgebenden Gewebe Polarisationseffekte (Umladungseffekte an der Grenzschicht zw. Elektrodenoberfläche und dem Elektrolyten), die zu einem Sprung der Amplitude des Eingangssignals der Wahrnehmungseinheit führen können, wenn diese nach der Stimulation wieder mit der Elektrode verbunden wird. Insbesondere Filter der Wahrnehmungseinheit reagieren auf diesen Sprung der Amplitude des Eingangssignals mit einer Sprungantwort, die sich als länger andauernde erhöhte Amplitude des Ausgangssignals der Wahrnehmungseinheit manifestiert und auch als Stimulationsartefakt bezeichnet wird. Solche Stimulationsartefakte können die Funktion des Gewebestimulators negativ beeinflussen, weil ein Stimulationsartefakt fälschlicherweise als natürliche elektrische Gewebeaktivität fehl interpretiert werden kann. Daher werden im Stand der Technik die Ausgangssignale der Wahrnehmungseinheit für eine bestimmte Zeitdauer nach der Stimulation von der Signalerkennung ausgeschlossen, was auch als digitales Blanking bezeichnet wird. Eine derartige Wahrnehmungseinheit ist z. B. aus Dokument US 2007/0146189 A1 bekannt.

Nachteilig daran ist, dass tatsächliche natürliche Aktivitäten des Körpergewebes während der Zeitdauer des digitalen Blanking nicht erkannt werden können.

Die vorliegende Erfindung löst die Aufgabe, eine Wahrnehmungseinheit zu schaffen, die diesen Nachteil überwindet und die eine frühere Signalerkennung nach einer Stimulation ermöglicht.

Üblicherweise werden zeitkontinuierliche analoge Signale bei der A/D-Wandlung in der Wahrnehmungseinheit mit einer bestimmten Abtastfrequenz abgetastet und in digital codierte zeit- und wertdiskrete Abtastwerte umgewandelt. Die Abtastfrequenz wird dem A/D-Wandler als Taktfrequenz zugeführt. Mit jedem Taktschritt der Taktfrequenz stellt der A/D-Wandler einen neuen Abtastwert bereit. Bei der nachfolgenden digitalen Filterung werden die Abtastwerte gemäß dem gewählten Filteralgorithmus mathematischen Operationen wie Multiplikation und/oder Addition mit Filterkoeffizienten unterworfen, wobei die gleiche Taktfrequenz wie bei der A/D-Wandlung verwendet wird. Das digitale Filter liefert somit mit jedem Taktschritt für den am Eingang vorliegenden Abtastwert einen modifizierten Abtastwert am Ausgang.

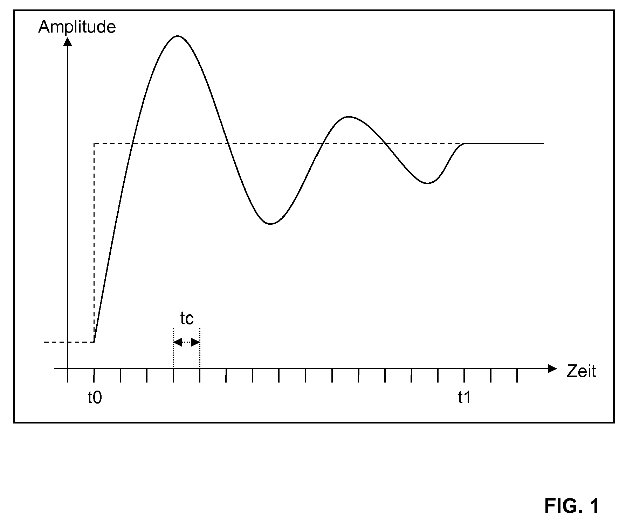

Digitale Filter reagieren auf eine sprunghafte Änderung der Amplitude des Eingangssignals prinzipbedingt mit einem in Figur 1 beispielhaft dargestellten Ausgangssignal. Der Amplitude des Filtereingangssignals ist als gestrichelte Linie dargestellt und erfährt zum Zeitpunkt t0 eine sprunghafte Änderung. Das als durchgehende Linie dargestellte Ausgangssignal des Filters ist gekennzeichnet durch ein zeitverzögertes Ansteigen der Amplitude auf einen Wert, der über der Amplitude des Eingangssignals liegt und sich danach der Amplitude des Eingangssignals zum Zeitpunkt t1 wieder annähert. Die in Figur 1 dargestellte Oszillation des Filterausgangssignals wird als Ausgleichsvorgang oder auch als Einschwingen des Filters bezeichnet, die Zeitdauer t1-t0 als Einschwingzeit, die sich über eine bestimmte Anzahl von Taktschritten tc, im dargestellten Beispiel 14 Taktschritte tc, erstreckt. Die Erfindung basiert auf der Erkenntnis, dass dieser Ausgleichsvorgang beziehungsweise die Einschwingzeit verkürzt werden kann, indem die Taktfrequenz des Filters für den Zeitraum nach einem Amplitudensprung am Eingang auf einen Wert vergrößert wird, der ein mehrfaches über der Taktfrequenz des A/D-Wandlers liegt. Die den Ausgleichsvorgang oder für das Einschwingen des Filters benötigte Anzahl von Taktschritten wird so in einer kürzeren Zeitdauer durchlaufen und das Ausgangssignal des Filters kann früher zur Signalerkennung verwendet werden.

Eine erfindungsgemäße Wahrnehmungseinheit für einen Gewebestimulator umfasst einen A/D-Wandler, der ein analoges Signal mit einem Abtasttakt abtastet und in ein digitales Signal umwandelt und ein digitales Filter, dessen Eingang mit dem Ausgang des A/D-Wandlers verbunden ist und das mit einem Filtertakt eine Filterung des digitalen Signals vornimmt, wobei für einen bestimmten Zeitraum T der Filtertakt ein Mehrfaches des Abtasttaktes beträgt.

Bevorzugt ist der Zeitraum T einstellbar. In einer Ausführungsform beginnt der Zeitraum T mit dem Ende der Trennung der Wahrnehmungseinheit von der Elektrode während und nach einem Stimulationsimpuls. Der Filtertakt während des Zeitraumes T beträgt bevorzugt das Doppelte bis Zehnfache des Abtasttaktes.

In einer weiteren Ausführungsform durchläuft das analoge Signal vor der Abtastung einen Vorverstärker und ein analoges Filter.

Bevorzugt ist die Wahrnehmungseinheit Bestandteil eines Gewebestimulators, wie eines Herzschrittmachers, Defibrillators, Kardioverters oder Neurostimulators, die auch als implantierbare Geräte ausgebildet sein können.

Ein solcher Gewebestimulator umfasst neben der Wahrnehmungseinheit mindestens einem Elektrodenanschluss, der über einen Schalter mit der Wahrnehmungseinheit und direkt mit einer Stimulationseinheit verbunden ist, einem Taktgeber, der einen Abtasttakt und einen Filtertakt bereitstellt und eine Steuereinheit, die mit Schalter, Wahrnehmungseinheit, Stimulationseinheit und Taktgeber wirkverbunden ist.

Die Steuereinheit des Gewebestimulators ist weiterhin ausgebildet für mindestens die Zeitdauer der Abgabe eines oder mehrer Stimulationsimpulse ein Öffnen des Schalters zu veranlassen, während außerhalb dieser Zeitdauer der Schalter geschlossen ist.

Weiterhin ist die Steuereinheit ausgebildet den Taktgeber für einen bestimmten Zeitraum T nach dem wieder Schließen des Schalters zu veranlassen, den Filtertakt zu erhöhen und den Abtasttakt beizubehalten..

Die Erfindung ist in Anspruch 1 definiert. Weitere bevorzugte Ausführungsbeispiele sind in den abhängigen Ansprüchen definiert.

Die Erfindung soll nun anhand eines Ausführungsbeispiels mit Bezug auf die Figuren näher erläutert werden.

Von den Figuren zeigen:
Figur 1: den Amplitudenverlauf des Ausgangssignales eines digitalen Filters
Figur 2: einen Herzstimulator
Figur 3: den schematischen Aufbau eines Herzstimulators

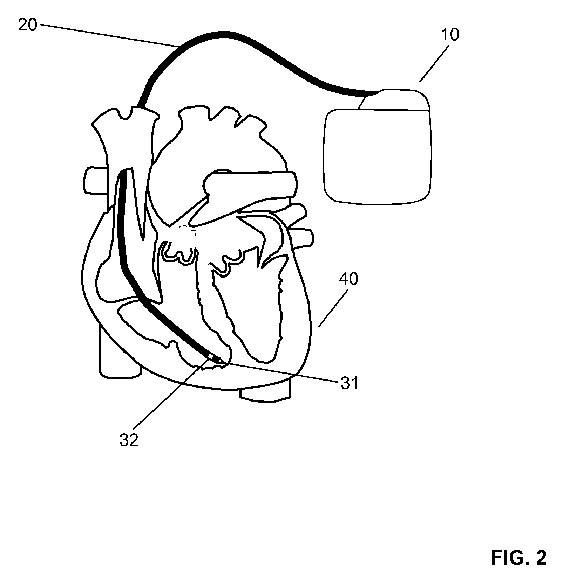

Figur 2 zeigt einen Herzstimulator (10), der über einen Elektrodenanschluss mit einer elektrisch leitenden isolierten Elektrodenleitung (20) verbunden ist. Das distale Ende der Elektrodenleitung weist eine oder mehrere Elektroden (31, 32) zur Aufnahme elektrischer Signale des Herzens (40) auf.

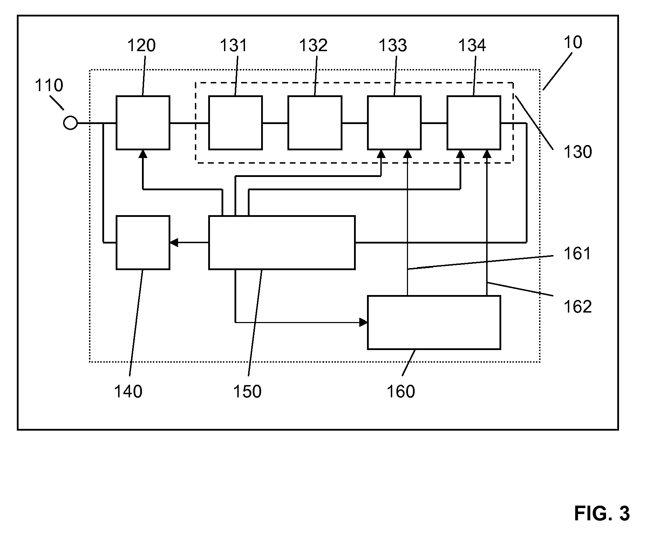

Figur 3 zeigt den weiteren Aufbau des Herzstimulators (10).

Der Herzstimulator beinhaltet eine Wahrnehmungseinheit (130) und eine Stimulationseinheit (140), die mit einer Steuereinheit (150) verbunden sind und durch diese gesteuert werden. Der Eingang der Wahrnehmungseinheit (130) ist über einen von der Steuereinheit (150) gesteuerten Schalter (120) mit dem Elektrodenanschluss (110) verbunden. Der Ausgang der Stimulationseinheit (140) ist ebenfalls mit dem Elektrodenanschluss (110) verbunden.

Weiterhin beinhaltet der Herzstimulator in Figur 3 nicht dargestellte eine elektrische Energiequelle, beispielsweise in Form einer Batterie oder eines Akkumulators, die Wahrnehmungseinheit, Stimulationseinheit und Steuereinheit mit Energie versorgt.

Der Herzstimulator beinhaltet außerdem einen Taktgeber (160), der Taktsignale bereitstellt und durch die Steuereinheit (150) steuerbar ist.

Die Wahrnehmungseinheit (130) erfasst die mit der Elektrode aufgenommenen elektrischen Signale des Herzens und wertet diese zur Erkennung physiologischer Ereignisse aus. Beispielsweise werden elektrische Signale von einer im Ventrikel platzierten Elektrode oberhalb eines bestimmten Amplitudenschwellwertes als natürliche Kontraktion des Ventrikels erkannt und an die Steuereinheit weitergegeben. Die Steuereinheit (150) kann beim Ausbleiben einer natürlichen Kontraktion des Ventrikels die Stimulationseinheit (140) veranlassen, einen oder mehrere elektrische Impulse zur Stimulation des Herzens abzugeben. Während der Abgabe eines Stimulationsimpulses veranlasst die Steuereinheit das Öffnen des ansonsten geschlossenen Schalters (120), womit die Wahrnehmungseinheit (130) elektrisch von Elektrodenanschluss (110) getrennt wird. Nach der Abgabe des Stimulationsimpulses veranlasst die Steuereinheit (150) das Schließen des Schalters (120), wodurch die Wahrnehmungseinheit (130) wieder mit dem Elektrodenanschluss (110) verbunden wird. Der Schalter (120) wird auch als Blankingschalter bezeichnet. Das Trennen der Wahrnehmungsstufe (130) vom Elektrodenanschluss (110) wird auch als analoges Blanking bezeichnet.

Dieser grundsätzliche Aufbau und weitere Ausgestaltungen eines Herzstimulators sind aus dem Stand der Technik hinreichend bekannt. Die Wahrnehmung elektrischer Signale des Herzens und die Stimulation kann, wie in Figur 2 dargestellt über zwei Elektroden am distalen Ende der Elektrodenleitung erfolgen und wird als bipolare Signalerfassung bzw. bipolare Stimulation bezeichnet. Alternativ kann auch eine unipolare Signalerfassung bzw. Stimulation erfolgen, bei der die Elektrodenleitung am distalen Ende nur eine Elektrode aufweist und das elektrisch leitende Gehäuse des Herzstimulators die zweite Elektrode ersetzt. Ebenso kann der Herzstimulator mehrere Wahrnehmungs- und Stimulationseinheiten aufweisen.

Im Folgenden wird auf den Aufbau der erfindungsgemäßen Wahrnehmungseinheit (130) von Figur 3 eingegangen.
Der Eingang der Wahrnehmungseinheit (130) ist über den Blankingschalter (120) mit dem Elektrodenanschluss (110) verbunden. Das von der Elektrode aufgenommene elektrische Signal durchläuft einen Vorverstärker (131) und ein analoges Filter (132) und wird anschließend mit einem A/D-Wandler (133) in ein digitales Signal umgesetzt. Der A/D-Wandler (133) enthält weiterhin einen Eingang für den für die A/D Wandlung notwendigen Abtasttakt (161), welcher vom Taktgeber (160) bereitgestellt wird durch eine Abtastfrequenz gekennzeichnet ist. In einer nicht dargestellten alternativen Ausführungsform durchläuft das von der Elektrode aufgenommene elektrische Signal zuerst das analoge Filter und wird dann über den Vorverstärker dem A/D-Wandler zugeführt. Der analoge Vorverstärker (131) und das analoge Filter (132) sind für die Ausführung der Erfindung nicht zwingend erforderlich und können gegebenenfalls auch entfallen. Das digitale Signal am Ausgang des A/D-Wandlers wird nach jedem Taktschritt auf den zeit- und wertdiskreten Abtastwert des analogen Eingangssignales aktualisiert anschließend mit einem digitalen Filter (134) gefiltert. Auch das digitale Filter hat einen Eingang für einen Filtertakt (162), welcher ebenfalls vom Taktgeber (160) bereitgestellt wird und durch eine Filtertaktfrequenz gekennzeichnet ist. Vom Ausgang des digitalen Filters (134) wird das Ausgangssignal der Wahrnehmungseinheit (130) einer hier nicht dargestellten Signalverarbeitungseinheit zur weiteren Verarbeitung zugeführt. Der Ausgang der Signalverarbeitungseinheit ist mit der Steuereinheit (150) verbunden. Alternativ kann die Signalverarbeitungseinheit auch Bestandteil der Steuereinheit (150) sein.

Die Arbeitsweise der Wahrnehmungseinheit (130) ist wie folgt:
Solange durch die Stimulationseinheit (140) kein Stimulationsimpuls abgegeben wird, ist der Blankingschalter (120) geschlossen. A/D-Wandler (133) und das digitale Filter (134) erhalten vom Taktgeber (160) einen identischen Abtasttakt und Filtertakt mit der gleichen Abtastfrequenz und Filtertaktfrequenz. Während der Abgabe eines Stimulationsimpulses wird der Blankingschalter (120) geöffnet und nach der Abgabe eins Stimulationsimpulses zum Ende der programmierbaren analogen Blankingzeit wieder geschlossen. Mit dem erneuten Schließen des Blankingschalters (120) wird durch den Taktgeber der Filtertakt für das digitale Filter (134) für eine bestimmte Anzahl von Takten erhöht, und anschließend wieder auf den ursprünglichen Wert reduziert, während der Abtasttakt für den A/D-Wandler (133) unverändert bleibt. Alternativ kann der Filtertakt für das digitale Filter (134) direkt nach Abgabe eines Stimulationspulses erhöht werden. Das Filter mit der in Figur 1 dargestellten Einschwingverhalten benötigt 14 Taktschritte nach dem Schließen des Blankingschalters für den Ausgleichsvorgang, was bei einem A/D-Wandler mit einer Abtastfrequenz von 500 Hz und einer Filtertaktfrequenz von 500 Hz zu einer Zeitdauer von 28 Millisekunden für das digitale Blanking führt. Dies bedeutet dass innerhalb von 28 Millisekunden nach einem Stimulationsimpuls keine Körpersignale detektiert werden können. Wird nun erfindungsgemäß die Filtertaktfrequenz für die ersten 14 Taktschritte nach dem Schließen des Blankingschalters um beispielsweise das 8-fache auf 4000 Hz vergrößert, ist der Ausgleichsvorgang bereits nach 3,5 Millisekunden abgeschlossen, so dass das digitale Blanking deutlich verkürzt werden kann.

## Patentansprüche

1. Wahrnehmungseinheit (130) für einen Gewebestimulator (10), umfassend einen A/D-Wandler (133), der ein analoges Signal mit einem Abtasttakt (161) abtastet und in ein digitales Signal umwandelt,
ein digitales Filter (134), dessen Eingang mit dem Ausgang des A/D-Wandlers verbunden ist und das mit einem Filtertakt (162) eine Filterung des digitalen Signals vornimmt,
**dadurch gekennzeichnet,**
**dass** für einen bestimmten Zeitraum T der Filtertakt (162) ein Mehrfaches des Abtasttaktes (161) beträgt.

2. Wahrnehmungseinheit (130) nach Anspruch 1,
**dadurch gekennzeichnet**
**dass** der Zeitraum T einstellbar ist.

3. Wahrnehmungseinheit (130) nach Anspruch 1 oder 2
**dadurch gekennzeichnet**
**dass** der Zeitraum T mit dem Ende eines Stimulationsimpulses beginnt.

4. Wahrnehmungseinheit (130) nach einem der vorgehenden Ansprüche,
**dadurch gekennzeichnet**
**dass** der Filtertakt (162) während des Zeitraumes T das Doppelte bis Zehnfache des Abtasttaktes (161) beträgt.

5. Wahrnehmungseinheit (130) nach einem der vorgehenden Ansprüche,
**dadurch gekennzeichnet**
**dass** das analoge Signal vor der Abtastung einen Vorverstärker (131) und ein analoges Filter (132) durchläuft.

6. Gewebestimulator (10) mit einer Wahrnehmungseinheit (130) nach einem der vorgehenden Ansprüche,
sowie mindestens einem Elektrodenanschluss (110) der über einen Schalter (120) mit der Wahrnehmungseinheit (130) und direkt mit einer Stimulationseinheit (140) verbunden ist,
einem Taktgeber (160), der ausgebildet ist einen Abtasttakt (161) und einen Filtertakt (162) bereitzustellen und
einer Steuereinheit (150), die mit Schalter (120), der Wahrnehmungseinheit (130), der Stimulationseinheit (140) und dem Taktgeber (160) wirkverbunden ist.

7. Gewebestimulator (10) nach Anspruch 6,
**dadurch gekennzeichnet**
**dass** die Steuereinheit (150) ausgebildet ist, für die Zeitdauer der Abgabe eines oder mehrer Stimulationsimpulse ein Öffnen des Schalters (120) zu veranlassen, während außerhalb dieser Zeitdauer der Schalter (120) geschlossen ist.

8. Gewebestimulator (10) nach Anspruch 7,
**dadurch gekennzeichnet**
**dass** die Steuereinheit (150) den Taktgeber (160) für einen bestimmten Zeitraum T nach dem Öffnen des Schalters (120) veranlasst, den Filtertakt (162) zu erhöhen und den Abtasttakt (161) beizubehalten.

9. Gewebestimulator (10) nach einem der Ansprüche 6 bis 8
**dadurch gekennzeichnet**
**dass** der Gewebestimulator als Herzschrittmacher, Defibrillator, Kardioverter oder Neurostimulator ausgebildet ist.

10. Gewebestimulator (10) nach Anspruch 9
**dadurch gekennzeichnet**
**dass** der Gewebestimulator als implantierbares Gerät ausgebildet ist.

## Claims

1. A sensing unit (130) for a tissue stimulator (10), comprising
an A/D converter (133), which samples an analogue signal by means of a sampling clock (161) and converts it into a digital signal,
a digital filter (134), of which the input is connected to the output of the A/D converter and which, by means of a filter clock (162), filters the digital signal,
**characterised in that**
the filter clock (162) is a multiple of the sampling clock (161) for a specific period of time T.

2. The sensing unit (130) according to claim 1,
**characterised in that**
the period of time T is adjustable.

3. The sensing unit (130) according to claim 1 or 2
**characterised in that** the period of time T starts with the end of a stimulation pulse.

4. The sensing unit (130) according to any one of the preceding claims,
**characterised in that**
the filter clock (162) is twice to ten times the sampling clock (161) during the period of time T.

5. The sensing unit (130) according to any one of the preceding claims,
**characterised in that**
the analogue signal, before sampling, passes through a preamplifier (131) and an analogue filter (132).

6. A tissue stimulator (10) having a sensing unit (130) according to any one of the preceding claims,
and also at least one electrode connection (110), which is connected via a switch (120) to the sensing unit (130) and directly to a stimulation unit (140),
a clock generator (160), which is designed to provide a sampling clock (161) and a filter clock (162), and
a control unit (150), which is operatively connected to the switch (120), the sensing unit (130), the stimulation unit (140), and the clock generator (160).

7. The tissue stimulator (10) according to claim 6,
**characterised in that**
the control unit (150) is designed to prompt an opening of the switch (120) for the period of the delivery of one or more stimulation pulses, whereas the switch (120) is closed outside this period.

8. The tissue stimulator (10) according to claim 7,
**characterised in that**
the control unit (150) prompts the clock generator (160) to increase the filter clock (162) and to maintain the sampling clock (161) for a specific period of time T once the switch (120) has been opened.

9. The tissue stimulator (10) according to any one of claims 6 to 8,
**characterised in that**
the tissue stimulator is designed as a cardiac pacemaker, defibrillator, cardioverter or neurostimulator.

10. The tissue stimulator (10) according to claim 9,
**characterised in that**
the tissue stimulator is designed as an implantable device.

## Revendications

1. Unité de détection (130) d'un stimulateur de tissu, comprenant
un convertisseur analogique/numérique (133), qui effectue un balayage d'un signal analogique avec une horloge d'échantillonnage (161) et le convertit en un signal numérique,
un filtre numérique (134) dont l'entrée est reliée avec la sortie du convertisseur analogique/numérique et qui effectue un filtrage du signal numérique avec une horloge de filtre (162),
**caractérisée en ce**
**que** pour un laps de temps T déterminé, l'horloge de filtre (162) est un multiple de l'horloge d'échantillonnage (161).

2. Unité de détection (130) selon la revendication 1,
**caractérisée en ce**
**que** pour le laps de temps peut être réglé.

3. Unité de détection (130) selon la revendication 1, ou 2,
**caractérisée en ce**
**que** le laps de temps commence à la fin d'une impulsion de stimulation.

4. Unité de détection (130) selon l'une des revendications précédentes,
**caractérisée en ce**
**que** l'horloge de filtre (162) est entre le double jusqu'à dix fois égal à l'horloge d'échantillonnage (161) pendant le laps de temps T.

5. Unité de détection (130) selon l'une des revendications précédentes,
**caractérisée en ce**
**que** le signal analogique passe par un préamplificateur (131) et un filtre analogique (132) avant le balayage.

6. Stimulateur de tissu (10) avec une unité de détection (130) selon l'une des revendications précédentes,
ainsi qu'au moins une borne d'électrode (110) qui est reliée avec l'unité de détection (130) par l'intermédiaire d'un commutateur (120) et est directement relié avec une unité de stimulation (140),
une horloge (160), qui est conçue pour fournir une horloge d'échantillonnage (161) et une horloge de filtre (162) et
une unité de commande (150), qui est reliée de manière opérationnelle avec le commutateur (120), l'unité de détection (130), l'unité de stimulation (140) et l'horloge (160).

7. Stimulateur de tissu (10) selon la revendication 6,
**caractérisé en ce**
**que** l'unité de commande (150) est conçue pour provoquer une ouverture du commutateur (120) pendant le laps de temps de la délivrance d'une ou plusieurs impulsions de stimulation, tandis que le commutateur (120) est fermé pendant ce laps de temps.

8. Stimulateur de tissu (10) selon la revendication 7,
**caractérisé en ce**
**que** l'unité de commande (150) fait en sorte que l'horloge de filtre (162) s'élève et l'horloge d'échantillonnage (161) est maintenue pendant un laps de temps T déterminé après l'ouverture du commutateur (120).

9. Stimulateur de tissu (10) selon l'une des revendications 6 à 8,
**caractérisé en ce**
**que** le stimulateur de tissu est conçu sous la forme d'un stimulateur cardiaque, d'un défibrillateur, d'un cardioverteur ou d'un neurostimulateur.

10. Stimulateur de tissu (10) selon la revendication 9,
**caractérisé en ce**
**que** le stimulateur de tissu est conçu sous la forme d'un appareil implantable.
